# EUROPEAN PATENT APPLICATION

(11) **EP 3 011 997 A1**
(43) Date of publication of application: **27.04.2016**
(21) Application number: 15155443.3
(22) Date of filing: 17.02.2015
(51) Int. Cl.: A61Q 3/00, A61Q 3/02, A61Q 3/04, A61K 8/81, A61K 8/87, A61K 8/02

(54) **Composition for nail polish removal and kit for nail polish removal including the same**

(30) Priority: 21.10.2014 KR 20140142786
(71) Applicant: JC Korea Corp., Gyeonggi-do (KR)
(72) Inventor: Kim, Hyun Surk, Gyeonggi-do (KR); Choi, Kyung Sik, Gyeonggi-do (KR); Park, Ju Young, Gyeonggi-do (KR); Choi, Jeong Rim, Gyeonggi-do (KR); Kim, Bo Mi, Seoul (KR)
(74) Representative: V.O.

(57) **Abstract**

A composition for nail polish removal is disclosed. The composition for nail polish removal is applied to a nail to form a layer for nail polish removal, and then a UV gel is applied to the layer for nail polish removal or an acrylic resin is applied to the layer for nail polish removal to extend the nail. Therefore, the UV gel or the acrylic resin can be peeled off in a simple manner without the use of a special tool or the need to perform a special operation. In addition, the risk of damage to the nail can be greatly reduced. Also disclosed is a kit for nail polish removal including the composition.

## Description

### Field of the Invention

The present invention relates to a composition for nail polish removal and a kit for nail polish removal including the composition.

### Description of the Related Art

The beauty industry has been motivated by the human desire to express their physical beauty. The beauty industry has developed rapidly and been subdivided into various branches of industry. Along with the popularization of the beauty industry, the market for the beauty industry has been expanding rapidly, particularly since the 20th century. Nail art, one of the beauty industries, is an essential element in the art of expressing the human body irrespective of age and sex. In recent years, remarkable progress has been made in the development of nail art.

Nail art was originally considered a field of beauty art to adorn the human body. Nails have reflected contemporary cultural changes and values by their length, shape, and color. Nail art is performed using various kinds of materials. Ultraviolet (UV) gels are attracting attention as promising materials for nail art. UV light is used to cure UV gels. UV gels are resistant to embrittlement due to their flexibility and softness. In addition, UV gels are hardly discolored even when exposed to sunlight and are very glossy. Such a UV gel is prepared by applying a UV gel composition to a natural nail to form a UV gel layer and curing the UV gel layer under UV irradiation. UV gels render natural nails glossy and shiny in appearance. Such attractive appearance makes UV gels useful in a wide range of applications.

Despite these advantages, removal of conventional UV gel layers directly formed on natural nails requires dipping of fingers in toxic solvents such as acetone for a long time. This dipping is very harmful to the skin and nails and causes severe damage to the natural nails after removal of the UV gel layers.

After a UV gel for nail art is applied to a nail or a suitable resin such as an acrylic resin is used to extend a nail, it is removed by surrounding the nail with a piece of gauze soaked with an organic solvent such as acetone for 5 to 10 minutes and detaching the gauze from the nail. The exposure to the organic solvent does severe damage to the nail and the end of the nail opposite the cuticle may be chipped or the end portion of the nail close to the cuticle is likely to fall off.

### Prior Art Documents

### Patent Documents

Korean Patent Publication No. 10-2012-0110844
Korean Patent Publication No. 10-2010-0125859
Korean Patent Publication No. 10-2014-0086437
Korean Patent Publication No. 10-2010-0108555

### Summary of the Invention

As a result of intensive research in view of the above problems, the inventors have found that when a composition for nail polish removal is applied to a natural nail to form a layer and a UV gel is applied to the layer or a suitable resin such as an acrylic resin is applied to the layer to extend the nail, the attractive appearance of the UV gel layer remains intact while maintaining the inherent advantages of the UV gel or the effect of nail extension by the resin remains unchanged. The inventors have also found that the UV gel can be easily peeled off without using any toxic organic solvents.

According to one aspect of the present invention, there is provided a composition for nail polish removal including (a) a polymer solution selected from a polyurethane solution, an aqueous solution of polyvinyl alcohol, a poly(meth)acrylate solution, and mixtures thereof.

According to a further aspect of the present invention, there is provided a kit for nail polish removal including (I) a composition for nail polish removal and (II) an instruction manual on how to use the composition for nail polish removal.

According to another aspect of the present invention, there is provided a method for nail care including (A) applying a composition for nail polish removal to a nail to form a layer for nail polish removal and (B) forming a nail polish layer on the layer for nail polish removal.

According to exemplary embodiments of the present invention, the composition for nail polish removal is applied to a nail to form a layer for nail polish removal, and then a UV gel is applied to the layer for nail polish removal or an acrylic resin is applied to the layer for nail polish removal to extend the nail. Therefore, the UV gel or the acrylic resin can be peeled off in a simple manner without the use of a special tool or the need to perform a special operation. In addition, the risk of damage to the nail can be greatly reduced.

### Detailed Description of the Invention

Several aspects and various embodiments of the present invention will now be described in more detail.

One aspect of the present invention is directed to a composition for nail polish removal including (a) a polymer solution selected from a polyurethane solution, an aqueous solution of polyvinyl alcohol, a poly(meth)acrylate solution, and mixtures thereof.

The polyurethane solution is composed of 25 to 40% by weight of polyurethane and 60 to 75% by weight of water. The aqueous solution of polyvinyl alcohol is composed of 1 to 25% by weight of polyvinyl alcohol and 75 to 99% by weight of water. The poly(meth)acrylate solution is composed of 5 to 50% by weight of poly(meth)acrylate and 50 to 95% by weight of water.

According to one embodiment, the composition for nail polish removal may further include (b) a silicone or fluorinated slip agent. In this embodiment, the composition for nail polish removal includes 95 to 99.9% by weight of the polymer solution (a) and 0.01 to 5% by weight of the slip agent (b).

According to a further embodiment, the composition for nail polish removal may further include (c) an acrylic pressure-sensitive adhesive in addition to the components (a) and (b). In this embodiment, the composition for nail polish removal includes 76 to 95% by weight of the polymer solution (a), 0.01 to 5% by weight of the slip agent (b), and 0.01 to 20% by weight of the pressure-sensitive adhesive (c).

According to another embodiment, the composition for nail polish removal may further include (d) glycerin in addition to the components (a), (b), and (c). In this embodiment, the composition for nail polish removal includes 66 to 95% by weight of the polymer solution (a), 0.01 to 5% by weight of the slip agent (b), 0.01 to 20% by weight of the pressure-sensitive adhesive (c), and 0.1 to 10% by weight of the glycerin (d).

It is preferred that the components of the composition for nail polish removal and their contents are adjusted such that the time required to dry or volatize the composition until a coating is formed is in the range of 50 to 200 seconds. The requirement in terms of drying or volatilization time is met when the composition includes the components (a), (b), (c), and (d) in the amounts within the respective ranges defined above. If the drying or volatilization time is less than 50 seconds, thin strings like spider webs are formed when the composition is applied with a brush, making it difficult for the composition to form a clear layer for nail polish removal. Further, the composition has poor storage stability. For example, during storage in a container, the composition tends to harden around the entrance of the container as well as in the container. Meanwhile, if the drying or volatilization time exceeds 200 seconds, it takes a long time for nail care, causing much inconvenience in use. Further, the delayed drying impedes the formation of a layer for nail polish removal with uniform adhesive strength, making it difficult to uniformly remove an overlying nail polish in a subsequent step.

According to another embodiment, the composition for nail polish removal may further include (e) a thickener in addition to the components (a), (b), (c), and (d). In this embodiment, the composition for nail polish removal includes 65 to 95% by weight of the polymer solution (a), 0.01 to 5% by weight of the slip agent (b), 0.01 to 20% by weight of the pressure-sensitive adhesive (c), 0.1 to 10% by weight of the glycerin (d), and 0.1 to 1% by weight of the thickener (e).

According to another embodiment, the composition for nail polish removal may further include (f) a vitamin in addition to the components (a), (b), (c), (d), and (e). In this embodiment, the composition for nail polish removal includes 45 to 95% by weight of the polymer solution (a), 0.01 to 5% by weight of the slip agent (b), 0.01 to 20% by weight of the pressure-sensitive adhesive (c), 0.1 to 10% by weight of the glycerin (d), 0.1 to 1% by weight of the thickener (e), and 0.1 to 20% by weight of the vitamin (f).

In each of the compositions according to the foregoing exemplary embodiments, if the content of the polyurethane solution is less than the corresponding lower limit, there is a risk that the composition may easily fall off, unlike when the content of the polyurethane solution falls within the range defined above. Meanwhile, if the content of the polyurethane solution exceeds the corresponding upper limit, an overlying nail polish layer is not clearly removed as a whole and its portions remain unseparated, unlike when the content of the polyurethane solution falls within the range defined above. Specifically, the nail polish layer is only partially removed as if it is split into pieces and falls apart, and as a result, the nail polish layer may be difficult to completely remove.

In each of the compositions according to the foregoing exemplary embodiments, if the content of the silicone or fluorinated slip agent is less than the corresponding lower limit, thin strings like spider webs are formed when the composition is applied with a brush, making it difficult for the composition to form a clear layer for nail polish removal, unlike when the content of the slip agent falls within the range defined above. Further, an overlying nail polish layer is only partially removed as if it is split into pieces and falls apart, and as a result, the nail polish layer may be difficult to completely remove. Meanwhile, if the content of the slip agent exceeds the corresponding upper limit, it takes an excessively long time to dry the composition and an overlying nail polish may be easily chipped or fall off, unlike when the content of the slip agent falls within the range defined above.

In each of the compositions according to the foregoing exemplary embodiments, if the content of the pressure-sensitive adhesive is less than the corresponding lower limit, the adhesive strength of the urethane to the surface of an underlying nail becomes excessively high, making it difficult to peel the resulting layer later, and thin strings like spider webs are formed when the composition is applied with a brush, making it difficult for the composition to form a clear layer for nail polish removal. Meanwhile, if the content of the pressure-sensitive adhesive exceeds the corresponding upper limit, the adhesive strength between a layer composed of the composition and the surface of an underlying nail becomes excessively low, making it difficult to stably attach a nail polish layer to the layer composed of the composition, unlike when the content of the pressure-sensitive adhesive falls within the range defined above.

In each of the compositions according to the foregoing exemplary embodiments, if the glycerin content is less than the corresponding lower limit, thin strings like spider webs are formed when the composition is applied with a brush, making it difficult for the composition to form a clear layer for nail polish removal, unlike when the glycerin content falls within the range defined above. Meanwhile, if the glycerin content exceeds the corresponding upper limit, the adhesive strength between a layer composed of the composition and the surface of an underlying nail becomes excessively low, making it difficult to stably attach a polish layer to the layer composed of the composition, unlike when the glycerin content falls within the range defined above.

In each of the compositions according to the foregoing exemplary embodiments, if the thickener content is less than the corresponding lower limit, the viscosity of the composition becomes excessively low, unlike when the thickener content falls within the range defined above. Due to this low viscosity, the thickness of a layer formed on a nail by one-time application is small, and as a result, the adhesive strength of the layer is not uniform over the entire area, making it difficult to uniformly remove an overlying nail polish in a subsequent step. Meanwhile, if the thickener content exceeds the corresponding upper limit, the composition is applied to an excessively large thickness to form a layer for nail polish removal, and as a result, it requires a long time to dry, unlike when the thickener content falls within the range defined above. Further, the adhesive strength of the layer is not uniform, making it difficult to uniformly remove an overlying nail polish in a subsequent step.

In each of the compositions according to the foregoing exemplary embodiments, if the vitamin is present in an amount of less than the corresponding lower limit, its effect of enhancing the functions of keratin, a major component of nails, to improve the health of nails and cure the state of damaged nails is negligible, unlike when the vitamin content falls within the range defined above. Meanwhile, if the vitamin is present in an amount exceeding the corresponding upper limit, the adhesive strength between a layer composed of the composition and the surface of an underlying nail becomes excessively low, making it difficult to stably attach a nail polish layer to the layer composed of the composition, unlike when the vitamin content falls within the range defined above.

In one embodiment, the polyurethane solution is preferably composed of 25 to 40% by weight of polyurethane and 60 to 70% by weight of water. If the solid content of the polyurethane in the solution is less than the lower limit, there is a risk that an overlying nail polish may not be easy to remove, unlike when the solid content of the polyurethane in the solution falls within the range defined above. Meanwhile, if the solid content of the polyurethane in the solution exceeds the upper limit, an overlying nail polish may be easily chipped or fall off, unlike when the solid content of the polyurethane in the solution falls within the range defined above.

The aqueous solution of polyvinyl alcohol is preferably composed of 5 to 30% by weight of polyvinyl alcohol and 70 to 95% by weight of water. If the solid content of the polyvinyl alcohol in the aqueous solution is less than the lower limit, there is a risk that an overlying nail polish may not be easy to remove, unlike when the solid content of the polyvinyl alcohol in the aqueous solution falls within the range defined above. Meanwhile, if the solid content of the polyvinyl alcohol in the aqueous solution exceeds the upper limit, an overlying nail polish may be easily chipped or fall off, unlike when the solid content of the polyvinyl alcohol in the aqueous solution falls within the range defined above.

The poly(meth)acrylate solution is preferably composed of 25 to 40% by weight of poly(meth)acrylate and 60 to 70% by weight of water. If the solid content of the poly(meth)acrylate in the solution is less than the lower limit, there is a risk that an overlying nail polish may fall off or be chipped, unlike when the solid content of the poly(meth)acrylate in the solution falls within the range defined above. Meanwhile, if the solid content of the poly(meth)acrylate in the solution exceeds the upper limit, an overlying nail polish may be difficult to uniformly remove, unlike when the solid content of the poly(meth)acrylate in the solution falls within the range defined above.

Examples of silicone and fluorinated slip agents suitable for use in the present invention include polysiloxane-based slip agents, perfluoroalkyl-based slip agents, carboxylic acid salts, sulfonic acid salts, ammonium salts, polysaccharides, and acetylene-based slip agents. These slip agents may be used alone or as a mixture of two or more thereof. Examples of pressure-sensitive adhesives suitable for use in the present invention include acrylic pressure-sensitive adhesives, rubber-based pressure-sensitive adhesives, and urethane-based pressure-sensitive adhesives. These pressure-sensitive adhesives may be used alone or as a mixture of two or more thereof. Examples of thickeners suitable for use in the present invention include cellulosic thickeners and urethane-based thickeners. These thickeners may be used alone or as a mixture of two or more thereof. Examples of vitamins suitable for use in the present invention include vitamins B1, B2, B5, C, E, and H, and calcium. These vitamins may be used alone or as a mixture of two or more thereof.

The inventors have confirmed that the use of the composition for nail polish removal in which the components are present in the amounts within the respective ranges defined above can solve the frequent problems encountered in the use of conventional UV gel layers, i.e. chipping at the end opposite the cuticle or falling off in the end portion close to the cuticle.

A further aspect of the present invention is directed to a kit for nail polish removal including (I) a composition for nail polish removal and (II) an instruction manual on how to use the composition for nail polish removal.

According to one embodiment, the composition for nail polish removal (I) may be one of the compositions for nail polish removal according to the exemplary embodiments of the present invention.

According to a further embodiment, the kit for nail polish removal may further include (III) a nail polish composition.

The nail polish composition may be composed of (1) a base UV gel composition, (2) a UV gel composition, and (3) a top UV gel composition. The nail polish composition may include a single composition capable of performing the functions of both the compositions (1) and (2). The nail polish composition may be composed of a single composition capable of performing the functions of all three compositions (1), (2), and (3). The UV gel composition (2) may further include a colorant such as a dye or a pigment.

Another aspect of the present invention is directed to a method for nail care including (A) applying a composition for nail polish removal to a nail to form a layer for nail polish removal and (B) forming a nail polish layer on the layer for nail polish removal.

According to one embodiment, the composition for nail polish removal may be one of the compositions for nail polish removal according to the exemplary embodiments of the present invention.

According to a further embodiment, step (B) may include (B1) forming a base UV gel layer on the layer for nail polish removal, (B2) forming a UV gel layer on the base UV gel layer, and (B3) forming a top UV gel layer on the UV gel layer.

According to another embodiment, step (B2) may include (B2') applying a UV gel composition to the base UV gel layer and (B2") irradiating UV onto the UV gel composition.

According to another embodiment, the layer for nail polish removal may have a thickness of 10 to 80 µm. If the thickness of the layer for nail polish removal is smaller than the lower limit, the nail polish layer is not clearly removed but is only partially removed as if it is split into pieces and falls apart, and as a result, the nail polish layer may be difficult to completely remove, unlike when the thickness of the layer for nail polish removal falls within the range defined above. Further, the nail may be stained with the color of the polish applied to the surface of the layer for nail polish removal. Meanwhile, if the thickness of the layer for nail polish removal exceeds the upper limit, there is a risk that the polish may be chipped or fall off, unlike when the thickness of the layer for nail polish removal falls within the range defined above.

According to another embodiment, the layer for nail polish removal may have a strength of 0.3 kgf or more (2.94 kg*m/s2), for example, 0.3 to 3 kgf (2.94-29.4 kg*m/s2) as measured by ASTM D683. If the strength of the layer for nail polish removal is less than the lower limit, the nail polish layer is not clearly removed as a whole but its portions remain unseparated, unlike when the strength of the layer for nail polish removal falls within the range defined above. Specifically, the nail polish layer is only partially removed as if it is split into pieces and falls apart, and as a result, the nail polish layer may be difficult to completely remove. For the purpose of clarity and a concise description features are described herein as part of the same or separate embodiments. However, it will be appreciated that the scope of the invention may include embodiments having combinations of all or some of the features described.

## Claims

1. A composition for nail polish removal comprising (a) a polymer solution selected from a polyurethane solution, an aqueous solution of polyvinyl alcohol, a poly(meth)acrylate solution, and mixtures thereof wherein the polyurethane solution is composed of 25 to 40% by weight of polyurethane and 60 to 75% by weight of water, the aqueous solution of polyvinyl alcohol is composed of 1 to 25% by weight of polyvinyl alcohol and 75 to 99% by weight of water, and the poly(meth)acrylate solution is composed of 5 to 50% by weight of poly(meth)acrylate and 50 to 95% by weight of water.

2. The composition for nail polish removal according to claim 1, further comprising (b) a silicone or fluorinated slip agent, wherein the composition for nail polish removal comprises 95 to 99.9% by weight of the polymer solution (a) and 0.01 to 5% by weight of the slip agent (b).

3. The composition for nail polish removal according to claim 1 or 2, further comprising (b) a silicone or fluorinated slip agent and (c) an acrylic pressure-sensitive adhesive, wherein the composition for nail polish removal comprises 76 to 95% by weight of the polymer solution (a), 0.01 to 5% by weight of the slip agent (b), and 0.01 to 20% by weight of the pressure-sensitive adhesive (c).

4. The composition for nail polish removal according to any of claims 1-3, further comprising (b) a silicone or fluorinated slip agent, (c) an acrylic pressure-sensitive adhesive, and (d) glycerin, wherein the composition for nail polish removal comprises 66 to 95% by weight of the polymer solution (a), 0.01 to 5% by weight of the slip agent (b), 0.01 to 20% by weight of the pressure-sensitive adhesive (c), and 0.1 to 10% by weight of the glycerin (d).

5. The composition for nail polish removal according to any of claims 1-4, further comprising (b) a silicone or fluorinated slip agent, (c) an acrylic pressure-sensitive adhesive, (d) glycerin, and (e) a thickener, wherein the composition for nail polish removal comprises 65 to 95% by weight of the polymer solution (a), 0.01 to 5% by weight of the slip agent (b), 0.01 to 20% by weight of the pressure-sensitive adhesive (c), 0.1 to 10% by weight of the glycerin (d), and 0.1 to 1% by weight of the thickener (e).

6. The composition for nail polish removal according to any of claims 1-5, further comprising (b) a silicone or fluorinated slip agent, (c) an acrylic pressure-sensitive adhesive, (d) glycerin, (e) a thickener, and (f) a vitamin, wherein the composition for nail polish removal comprises 45 to 95% by weight of the polymer solution (a), 0.01 to 5% by weight of the slip agent (b), 0.01 to 20% by weight of the pressure-sensitive adhesive (c), 0.1 to 10% by weight of the glycerin (d), 0.1 to 1% by weight of the thickener (e), and 0.1 to 20% by weight of the vitamin (f).

7. The composition for nail polish removal according to any of claims 3-6, wherein the pressure-sensitive adhesive is selected from acrylic pressure-sensitive adhesives, rubber-based pressure-sensitive adhesives, urethane-based pressure-sensitive adhesives, and mixtures thereof, and the thickener is selected from cellulosic thickeners, urethane-based thickeners, and mixtures thereof.

8. A kit for nail polish removal comprising (I) a composition for nail polish removal and (II) an instruction manual on how to use the composition for nail polish removal.

9. The kit for nail polish removal according to claim 8, wherein the composition for nail polish removal (I) is the composition for nail polish removal according to any one of claims 1 to 7,
wherein the kit preferably further comprises (III) a nail polish composition.

10. The kit for nail polish removal according to any of claim 8 or 9, wherein the nail polish composition is composed of (1) a base UV gel composition, (2) a UV gel composition, and (3) a top UV gel composition.

11. A method for nail care comprising (A) applying a composition for nail polish removal to a nail to form a layer for nail polish removal and (B) forming a nail polish layer on the layer for nail polish removal.

12. The method for nail care according to claim 11, wherein the composition for nail polish removal is the composition for nail polish removal according to any one of claims 1 to 7.

13. The method for nail care according to claim 11 or 12, wherein step (B) comprises (B1) forming a base UV gel layer on the layer for nail polish removal, (B2) forming a UV gel layer on the base UV gel layer, and (B3) forming a top UV gel layer on the UV gel layer, wherein step (B2) preferably comprises (B2') applying a UV gel composition to the base UV gel layer and (B2") irradiating UV onto the UV gel composition.

14. The method for nail care according to any of claims 11-13, wherein the layer for nail polish removal has a thickness of 10 to 20 µm.

15. The method for nail care according to any of claims 11-14, wherein the layer for nail polish removal has a strength of 0.3 to 3 kgf (2.94-29.4 m*s2).
